# EUROPEAN PATENT SPECIFICATION

(11) **EP 0 314 338 B1**
(45) Date of publication and mention of the grant of the patent: **11.08.1993**
(21) Application number: 88309546.5
(22) Date of filing: 12.10.1988
(51) Int. Cl.: G01N 33/74, G01N 33/577

(54) **Method for measuring human insulin**
Verfahren zur Messung von humanen Insulin
Méthode de mesure de l'insuline humaine

(30) Priority: 12.10.1987 JP 254621/87
(43) Date of publication of application: 03.05.1989
(73) Proprietor: Tosoh Corporation, Shinnanyo-shi, Yamaguchi-ken, 746 (JP)
(72) Inventor: Inouye, Kuniyo, Sagamihara-Shi Kanagawa (JP); Satoh, Kazuki, Sakado-Shi Saitama (JP)
(74) Representative: Kearney, Kevin David Nicholas

(56) References cited:
- DE-A- 3 411 472
- CHEMICAL ABSTRACTS, vol. 103, no. 15, 14th October 1985, page 554, no. 121356z, Columbus, Ohio, US; M.J. STORCH et al.: "Recognition of human insulin and proinsulin by monoclonal antibodies", & DIABETES 1985, 34(8, Pt. 1), 808-11
- CHEMICAL ABSTRACTS, vol. 104, no. 11, 17th March 1986, page 511, no. 86756b, Columbus, Ohio, US; A. LIVNEH et al.: "A human monoclonal antibody to insulin", & DIABETES 1986, 35(1), 68-73
- CHEMICAL ABSTRACTS, vol. 101, no. 17, 22nd October 1984, page 534, no. 149476w, Columbus, Ohio, US; M. ZIEGLER et al.: "Monoclonal antibodies to human insulin and their antigen binding behavior", & BIOMED. BIOCHIM. ACTA 1984, 43(5), 695-701
- PROCEEDINGS OF BIOTECH 83, London, 4th-6th May 1983, pages 823-832; D. LOW: "Purification of monoclonal antibodies and their use in the isolation of biological products"

## Description

The present invention relates to a method of measuring human insulin in a sample by utilising a monoclonal antibody to human insulin. More particularly, the present invention relates to a method of measuring human insulin at a human insulin concentration ranging from 1 pg/ml to 1 ng/ml.

Insulin is a peptide hormone secreted from beta cells of Langerhans' islet present in the pancreas and has the function of adjusting the metabolism of saccharides, fats and proteins in tissues of the liver and other organs. If the production of insulin in the living body is insufficient, accumulation of saccharides in the form of glycogen in the liver becomes difficult, and as a result, the blood glucose abruptly increases and the absorption of glucose by the kidney cannot cope with this increase of the blood glucose, causing diabetes.

Accordingly, the measurement of the insulin content in the living body is very important for the diagnosis of diabetes. At present, immunoassay methods such as the radioimmunoassay method and the enzyme immunoassay method are adopted for the determination of human insulin, but since the anti-insulin antibody used in these immunoassay methods is an antiserum obtained by immunising an animal such as a guinea pig with bovine insulin or swine insulin, the amount of the anti-insulin antibody obtained is very small and the affinity to insulin or the titer is often different in individual animals. Accordingly, the antiserum is collected from animals and the thus-collected fractions are mixed to obtain a product having a uniform quality for the use of immunoassay. Nevertheless, a difference of the affinity or titer can be still observed among the individual lots.

As means for solving this problem, a method has recently been proposed in which insulin is detected by using a monoclonal antibody (see DE-A-3 411 472 02 Japanese Unexamined Patent Publication No. 60-57253). In this method, the affinity to insulin is measured by radioimmunoassay or double bond assay.

Furthermore, R. Committi et al. reported a method in which insulin is measured at a concentration ranging from 0.08 ng/ml to 7.5 ng/ml within 3 to 4 hours by an enzyme immunoassay utilising the sandwich method (see Journal of Immunological Methods, volume 99, pages 25 through 37). In this reported method, from monoclonal antibodies obtained by using swine insulin as the antigen, monoclonal antibodies capable of cross reaction to human insulin are selected. Furthermore, when constructing the sandwich immunoassay system, a first antibody is immobilised in a solid phase, and a second antibody bound with biotin and a sample liquid containing human insulin are added to the immobilised first antibody to form a first antibody/human insulin/second antibody/biotin complex. Then, an avidin-bonded alkaline phosphatase is added to form a first antibody/human insulin/second antibody/biotin/avidin/alkaline phosphatase complex, and a substrate for the alkali phosphatase is added to cause an enzyme reaction. It is taught that, in 71 first antibody/second antibody pairs disclosed in the report, and paired two of antibodies cannot be simultaneously bonded to the human insulin bonded to the solid phase.

The problem involved in the above-mentioned reports and prior art is that the antibody shows a cross reaction to not only human insulin but also insulins of other animals such as swine, bovine and sheep. Since monoclonal antibodies are obtained by immunising mice or rats with insulins of animals other than human beings, such as bovine and swine, in most of the prior art, it is quite obvious that the obtained monoclonal antibodies react with the insulins of the animals used as the antigen. Among these monoclonal antibodies, there have been found monoclonal antibodies having a gentle specificity and capable of cross reaction with human insulin, and in most of the prior art, monoclonal antibodies produced to swine or bovine insulin, which are capable of a cross reaction to human insulin, are used.

Japanese Unexamined Patent Publication No. 60-237362 discloses a method for measuring insulin by the sandwich assay, but a polyclonal antibody derived from capybara or guinea pigs is used.

An object of the present invention is to overcome the above-mentioned defects of the conventional methods and provide a method in which human insulin can be measured at a high sensitivity.

The inventors prepared monoclonal antibodies specific to human insulin to be measured and examined immunological measurement methods using these monoclonal antibodies, and, as a result, found that the prepared monoclonal antibodies can be very valuable as reagents for measuring human insulin. Two monoclonal antibodies capable of binding specifically to human insulin, which have a different antigen-deciding site and can be simultaneously and independently bound to human insulin without competition, are used. These monoclonal antibodies are selected to detect only human insulin and to be such as not to react with insulin derived from a heterogeneous animal such as swine, bovine, goat, sheep or mice. Detection of human insulin is desired to be possible in the order of 1 pg/ml.

In accordance with the present invention, there is provided a method of detecting and/or measuring human insulin in a sample by contacting a first antibody and a second antibody with the sample, one of the antibodies being immobilised and the other antibody being labelled with a marker, characterised in that both of the antibodies are capable of binding to human insulin but at different antigenic sites but are such as not to react with non-human insulin.

The monoclonal antibodies used in the present invention can be obtained from a cultured products of hybridomas fusing antibody-producing spleen cells, namely, lymphoid cells in the spleen of an animal, for example, a mouse which is immunised with human insulin, to myeloma cells to obtain hybridoma producing monoclonal antibodies recognising the insulin.

The hybridoma producing the monoclonal antibody recognising human insulin can be prepared by the known cell-fusing method (G. Kohler and C. Milstein, Nature, volume 256, page 495, 1975).

According to the present invention, the monoclonal antibodies recognising human insulin are obtained by the above-mentioned method. The monoclonal antibodies comprise two monoclonal antibodies recognising different antigenic sites of the insulin, and therefore, the solid phase enzyme immunoassay according to the sandwich method using two monoclonal antibodies can be employed. Furthermore, monoclonal antibodies capable of reacting only with human insulin but incapable of a cross reaction with insulin derived from a heterogeneous animal such as bovine, swine, goat, sheep, mice or rats can be obtained according to the above-mentioned method. It is thought that the monoclonal antibodies obtained recognise the peptide structure inherently possessed by human insulin but not possessed by a heterogeneous animal such as bovine, swine, goat, sheep, mouse or rat. By using these monoclonal antibodies as at least one of the first and second antibodies, human insulin can be detected alone without a detection of insulin derived from a heterogeneous animal.

In the present invention, the monoclonal antibody recognising insulin, as the first antibody, is immobilised, and the immobilisation can be accomplished by known methods. For the immobilisation of the antibody, preferably beads or microplates of polystyrene, polyethylene, polyvinyl chloride, latex, agarose, cellulose, methacrylate or glass can be used.

The method or means for labelling the second antibody is not particularly critical, and unknown methods or means can be adopted. In the method using an enzyme as the marker (EIA), enzymes such as peroxidase, beta-D-galactosidase and alkaline phosphatase can be used, and in the method using a radio-active substance (RIA), for example, ¹²⁵I and ³H can be used. Fluorescein isothiocyanate is usually used in the method using a fluorescent substance (FIA), but other markers can be used.

Where the marker is an enzyme, a substrate is used for measuring the activity. For example, as the substrate for horseradish peroxidase, there can be mentioned diammonium 2 , 2´ -azino-di-[3-ethylbenzthiazoline 6-sulfonic acid diammonium salt] (hereinafter referred to as "ABTS") -H₂O₂, 5-aminosalicylic acid-H₂O₂ and o-phenylenediamine-H₂O₂. As the substrate for beta-galactosidase, there can be mentioned o-nitrophenyl-beta-D-galactopyranoside, and as the substrate for alkali phosphatase, there can be mentioned p-nitrophenyl phosphate.

For the measurement, known reagents such as dissolving agents, washing agents and reaction stoppers can be used in addition to the above-mentioned reagents.

According to the present invention, an infinitesimal amount (1 pg/ml) of human insulin can be detected at a high sensitivity with a good reproducibility, in a measurement concentration range of from 1 pg/ml to 1 ng/ml. Furthermore, according to the present invention, only human insulin can be detected without a reaction with insulin derived from a heterogeneous animal.

The present invention will now be described in detail with reference to the following examples.

### Example (A) Preparation of the Monoclonal Antibodies to Human Insulin

The monoclonal antibodies to human insulin were prepared by the method of G. Kohler and C. Milstein. In phosphate-buffered saline was dissolved 100 µg of human insulin (supplied by Sigma Co.), and the solution was emulsified with an equal amount of Freund's complete adjuvant. The emulsion was injected into a mouse intraperitoneally. After 20 days, a solution of 100 µg of human insulin in phosphate-buffered saline was injected to the mouse intraperitoneally. After 3 days, spleen cells were collected from the mouse and fused with mouse myeloma cells by using polyethylene glycol. The cells were cultured on 96 wellplates, and HAT selection was carried out in a known manner. The hybridoma was screened by the known method using a 96-well microtiter plate. With respect to the hybridoma producing antibodies to human insulin, cloning was carried out according to the limiting dilution. The thus-obtained hybridomas were cultured in the abdominal cavity of a mouse to obtain the ascites fluids containing monoclonal antibodies.

A plurality of kinds of monoclonal antibodies to human insulin were obtained by subjecting the ascites fluids to ammonium sulphate precipitation and DEAE ionechange column chromatography.

### Example (B) Immobilisation of the Monoclonal Antibody to Insulin

60 µl of a solution of the monoclonal antibody to human insulin [the monoclonal antibody prepared in Example (A), capable of reacting with human insulin but incapable of reacting with insulin derived from a heterogeneous animal, referred to as "UMI"], dissolved at a concentration of 100 µg/ml in phosphate-buffered saline, was added to respective wells of a 96-well microtiter plate (Immunoplate supplied by NVNK) and allowed to stand at 37°C for 2 hours. Then, the solution was removed from each well and 200 µl of phosphate-buffered saline containing 1% of BSA (bovine serum albumin) was added to each well, and each well was allowed to stand for 1 hour to block non specific adsorption sites. The monoclonal antibody immobilised microtiter plate was stored in this state at 4°C.

### Example (C) Preparation of the Antibody Labelled with Horseradish Peroxidase (hereinafter referred to as "HRPO")

To an HRPO solution (5 mg/ml) in 0.3M sodium bicarbonate buffer (pH = 8.1) was added 0.1 ml of a 1% solution of 1-fluoro-2,4-dinitrobenzene in ethanol, and reaction was carried out at room temperature for 1 hour. Then, 1.0 ml of 60 mM sodium periodate was added to the reaction mixtures and reaction was carried out for 30 minutes. Unreacted sodium periodate was removed by addition of 1.0 ml of 0.16M ethylene glycol, and the reaction mixture was dialyzed against 10 mM sodium carbonate buffer
(pH = 9.5).

Then, 5 mg of the mouse monoclonal antibody to human insulin [monoclonal antibody prepared in Example (A), which recognised an antigenic site different from the antigenic site recognised by UMI and was capable of reacting with human insulin but incapable of reacting with insulin derived from a heterogeneous animal; referred to as "DEW"] was added to the dialysate, and reaction was carried out for 6 hours. Then, 5 mg of sodium borohydride was added to the reaction mixture, and the mixture was allowed to stand at 4°C overnight.

The thus-obtained reaction product was purified by the high-performance liquid chromatography using TSK Gel G-3000SW (tradename for the product supplied by TOSOH Corp.), whereby a monoclonal antibody labelled with HRPO was obtained.

### Example (D) Measurement of Human Insulin by Enzyme Immunoassay

The anti-human insulin monoclonal antibody immobilized microtiter plate prepared by the method described in Example (B) was returned to room temperature, and the microtiter plate was washed with phosphate buffered saline. Then, 50 µl of a solution of 1 pg/ml to 1 ng/ml of human insulin (supplied by Sigma Co.) in phosphate-buffered saline was added to each well.

Then, 50 µl of a solution obtained by diluting 1000 times 4.5 mg/ml of the HRPO -labelled antibody prepared in Example (C) with phosphate-buffered saline containing 0.01% of myoglobin, 0.075% of refined white sugar and 0.05% of egg albumin was added to each well, and each well was allowed to stand at room temperature for 3 hours. The solution was removed and each well was washed three times with phosphate-buffered saline. Then, 50 µl of a substrate solution consisting of 0.1M citrate buffer (pH = 4.1) containing 0.3 mg/ml of ABTS and 0.01% of H₂O₂ was added to each well and reaction was carried out at room temperature for 30 minutes. The reaction was stopped by an addition of 50 µl of 1 M citric acid. After the reaction was stopped, with respect to each well, the absorption intensity at a wavelength of 415 nm (reference wavelength = 492 nm) was measured by an automatic microtiter plate reader (MPR-A4 supplied by TOSOH Corp.). The results as shown in Table 1 were obtained, and a calibration curve was obtained by using these results.

**Table 1**

| Human Insulin Concentration (pg/ml) in Sample | Absorbance (415 nm) |
|---|---|
| 1 | 0.01 |
| 2 | 0.02 |
| 5 | 0.05 |
| 10 | 0.11 |
| 40 | 0.48 |
| 80 | 0.95 |
| 320 | 1.54 |
| 500 | 2.11 |
| 1000 | 2.46 |

From the foregoing results, it is seen that the method of the present invention is suitable for the microanalytic determination of human insulin in various samples.

### Comparative Example (E) Measurement of Bovine and Swine Insulins by Enzyme Immunoassay

The measurement was carried out by the enzyme immunoassay in the same manner as described in Example (D) except that 10 pg/ml or 1000 pg/ml of bovine insulin (supplied by Sigma Co.) or swine insulin (supplied by Sigma) was used as the sample. The results are shown in Tables 2 and 3.

**Table 2**

| Bovine Insulin Concentration (pg/ml) in Sample | Absorbance (415 nm) |
|---|---|
| 10 | 0.01 |
| 1000 | 0.02 |

**Table 3**

| Swine Insulin Concentration (pg/ml) in Sample | Absorbance (415 nm) |
|---|---|
| 10 | 0.01 |
| 1000 | 0.02 |

From the foregoing results it can be seen that the monoclonal antibodies used in the present Examples react with human insulin but do not react with insulins of heterogeneous animals.

## Claims

1. A method of detecting and/or measuring an amount of human insulin in a sample, the method comprising contacting a first antibody and a second antibody with the sample, one of the antibodies being immobilised and the other antibody being labelled with a marker, characterised in that both of the antibodies are capable of binding to human insulin but at different antigenic sites but are such as not to react with non-human insulin.

2. A method as claimed in Claim 1 characterised in that it comprises measuring an amount of human insulin present in a sample at a concentration of from 1 pg/ml to 1 ng/ml.

3. A method of measuring human insulin as claimed in Claim 1 or Claim 2 characterised in that the monoclonal antibody does not react with bovine, swine, goat, sheep, mouse or rat insulin.

4. A method as claimed in any one of Claims 1 to 3 characterized in that the immobilized antibody is attached to beads or microplatez of polystyrene, polyethylene, polyvinyl chloride, latex, agarose, cellulose, methacrylate or glass.

5. A monoclonal antibody characterized in that it is capable of binding to human insulin but such as not to react with non-human insulin.

6. An antibody as claimed in Claim 5 characterized in that the non-human insulin is bovine, swine, goat, sheep, mouse or rat insulin.

7. A process for the preparation of monoclonal antibody characterized in that it is capable of binding to human insulin but such as not to react with non-human insulin, the process comprising :
(a) immunising a mammal with human insulin;
(b) fusing a spleen cell from the mammal with a cancer cell to produce hybridoma cells;
(c) culturing the hybridoma cells; and
(d) selecting a hybridoma cell expressing antibody capable of binding to human insulin but such as not to react with non-human insulin.

8. A process as claimed in Claim 7 characterized in that the non-human insulin is bovine, swine, goat, sheep, mouse or rat insulin.

## Patentansprüche

1. Verfahren zum Nachweisen und/oder Messen einer Menge von Humaninsulin in einer Probe, wobei das Verfahren das In-Kontakt-Bringen eines ersten Antikörpers und eines zweiten Antikörpers mit der Probe umfaßt, wobei einer der Antikörper immobilisiert und der andere Antikörper mit einem Marker markiert ist, **dadurch gekennzeichnet**, daß beide Antikörper an Humaninsulin binden können, jedoch an verschiedenen antigenen Stellen und so sind, daß sie nicht mit Nicht-Humaninsulin reagieren.

2. Verfahren nach Anspruch 1, **dadurch gekennzeichnet**, daß es das Messen einer Menge von Humaninsulin umfaßt, das in einer Probe bei einer Konzentration von 1 pg/ml bis 1 ng/ml vorhanden ist.

3. Verfahren zum Messen von Humaninsulin nach Anspruch 1 oder Anspruch 2, **dadurch gekennzeichnet**, daß der monoklonale Antikörper nicht mit Rinder-, Schweine-, Ziegen-, Schaf-, Maus- oder Ratteninsulin reagiert.

4. Verfahren nach einem der Ansprüche 1 bis 3, **dadurch gekennzeichnet**, daß der immobilisierte Antikörper an Kugeln oder Mikroplatten aus Polystyrol, Polyethylen, Polyvinylchlorid, Latex, Agarose, Zellulose, Methacrylat oder Glas angeheftet ist.

5. Monoklonaler Antikörper, **dadurch gekennzeichnet**, daß er an Humaninsulin binden kann, aber so, daß er nicht mit Nicht-Humaninsulin reagiert.

6. Antikörper nach Anspruch 5, **dadurch gekennzeichnet**, daß das Nicht-Humaninsulin Rinder-, Schweine-, Ziegen-, Schaf-, Maus- oder Ratteninzulin ist.

7. Verfahren zum Herstellen eines monoklonalen Antikörpers, **dadurch gekennzeichnet**, daß er an Humaninsulin binden kann, aber so, daß er nicht mit Nicht-Humaninsulin reagiert, wobei das Verfahren umfaßt:
a) Das Immunisieren eines Säugetieres mit Humaninzulin;
b) Das Verschmelzen einer Milzzelle aus dem Säugetier mit einer Krebszelle zur Erzeugung von Hybridomzellen;
c) Das Kultivieren der Hybridomzellen; und
d) Das Auswählen einer Hybridomzelle, die einen Antikörper exprimiert, der an Humaninsulin binden kann, aber so, daß er nicht mit Nicht-Humaninsulin reagiert.

8. Verfahren nach Anspruch 7, **dadurch gekennzeichnet**, daß das Nicht-Humaninsulin Rinder-, Schweine-, Ziegen-, Schaf-, Maus- oder Ratteninsulin ist.

## Revendications

1. Méthode de détection et/ou de mesure d'une quantité d'insuline humaine dans un échantillon, la méthode consistant à mettre en contact un premier anticorps et un deuxième anticorps avec l'échantillon, l'un des anticorps étant immobilisé et l'autre anticorps étant marqué avec un marqueur, caractérisé en ce que les deux anticorps sont capables de se fixer à l'insuline humaine mais en des sites antigéniques différents mais ne sont pas capables de réagir avec l'insuline non humaine.

2. Méthode selon la revendication 1, caractérisée en ce qu'elle comprend la mesure d'une quantité d'insuline humaine présente dans un échantillon à une concentration de 1 pg/ml à 1 ng/ml.

3. Méthode de mesure de l'insuline humaine selon la revendication 1 ou la revendication 2, caractérisée en ce que l'anticorps monoclonal ne réagit pas avec l'insuline bovine, porcine, de la chèvre, du mouton, de la souris ou du rat.

4. Méthode selon l'une quelconque des revendications 1 à 3, caractérisée en ce que l'anticorps immobilisé est fixé sur des perles ou des microplaquettes de polystyrène, de polyéthylène, de polychlorure de vinyle, de latex, d'agarose, de cellulose, de méthacrylate ou de verre.

5. Anticorps monoclonal, caractérisé en ce qu'il est capable d'être fixé à l'insuline humaine mais qu'il n'est pas capable de réagir avec l'insuline non humaine.

6. Anticorps selon la revendication 5, caractérisé en ce que l'insuline non humaine est l'insuline bovine, porcine, de chèvre, de mouton, de souris ou de rat.

7. Procédé de préparation d'un anticorps monoclonal, caractérisé en ce qu'il est capable d'être fixé à l'insuline humaine mais n'est pas capable de réagir avec l'insuline non humaine, le procédé comprenant:
(a) l'immunisation d'un mammifère par l'insuline humaine;
(b) la fusion d'une cellule de rate provenant du mammifère avec une cellule cancéreuse pour produire des cellules d'hybridome;
(c) la culture des cellules d'hybridome; et
(d) la sélection d'une cellule d'hybridome exprimant un anticorps capable d'être fixé à l'insuline humaine mais incapable de réagir avec l'insuline non humaine.

8. Procédé selon la revendication 7, caractérisé en ce que l'insuline non humaine est l'insuline bovine, porcine, de chèvre, de mouton, de souris ou de rat.
